# EUROPEAN PATENT SPECIFICATION

(11) **EP 4 605 044 B1**
(45) Date of publication and mention of the grant of the patent: **23.09.2026**
(21) Application number: 23801510.1
(22) Date of filing: 11.10.2023
(51) Int. Cl.: A61M 15/00

(54) **BREATH ACTUATED INHALER**
ATMUNGSBETÄTIGTER INHALATOR
INHALATEUR ACTIONNÉ PAR LA RESPIRATION

(30) Priority: 17.10.2022 US 202263416832 P
(43) Date of publication of application: 27.08.2025
(73) Proprietor: Lupin Limited, Mumbai 400 055 (IN)
(72) Inventor: SHAIKH, Imran, Coral Springs, Florida 33065 (US); DALVI, Mukul, Coral Springs, Florida 33065 (US); GRIDER, Keith, Chicago, Illinois 60647 (US)
(74) Representative: HGF
(86) International application number: PCT/IB2023/060234
(87) International publication number: WO 2024/084342

(56) References cited:
- WO-A1-2018/149620
- WO-A2-01/93933
- US-B2- 10 792 447

## Description

### FIELD OF THE INVENTION

The present invention relates to a breath actuated metered dose inhaler. The invention further provides modified version of a diaphragm and a flap for a trigger mechanism of a breath actuated metered dose inhaler. The invention further provides methods for manufacturing the same.

### BACKGROUND TO THE INVENTION

There are a variety of inhalation devices which release aerosol medication, either in a continuous spray or in a predetermined amount. Examples of such inhalation devices includes pressurized metered-dose inhaler (pMDI) and dry-powder inhaler (DPI). Generally, pMDIs are comprised of three major components; an aerosol canister where the formulation resides for administration to the lungs, a metering valve which is disposed in the canister and which allows a metered quantity of the formulation to be dispensed with each actuation, an actuator which holds the canister and allows the patient to operate the device and directs the aerosol into the patient's lungs. Most common in this category are "press and breathe" type pMDI inhalation devices and these devices are actuated by the pressure applied by the user's fingers, button action, or other related manual techniques. In use a "puff" or single dose of the stored formulation is metered and delivered when the patient depresses the canister within the actuator.

A more recent development is the so-called "breath actuated inhaler" which delivers a dose of drug through a mouthpiece in response to inhalation by the user. Breath actuated inhalers are preferred in circumstances where the co-ordination between user inhalation and manual depression of the aerosol canister is imperfect. For example, children or elderly patients have difficulty in synchronising actuation of the MDI with inhalation or sometimes patient breathes out before inhalation is complete.

PCT application WO 01/93933 A2 describes such breath-actuated metered dose inhaler. A preload is applied to the internal aerosol valve by an amount sufficient to result in a dose release, but this is prevented by the application of a pneumatic resisting force. Inhalation causes the releases of the pneumatic resisting force and allows the preload to actuate the aerosol valve. The pneumatic resisting force is established by a negative pressure region defined in part by a diaphragm. The diaphragm includes a central disk of a first, relatively high stiffness material and a peripheral ring, coupled by a flexure of a second, relatively low stiffness material. The diaphragm also includes a small valve port which is covered by a valve seal (flap). A valve seal (flap) seals a valve port to create a pressure differential. On inhalation by the patient through the mouthpiece, the valve seal (flap) moved out of a sealing position to open the valve port to break the pressure differential causing the preload to actuate the aerosol valve.

U.S. Pat. No. 10,792,447 B2 describes a more recent breath-actuated metered dose inhaler. The patent discloses a modified structure of the valve port of the pneumatic force holding unit which purportedly reduces the rate of degradation of the pressure difference within a pneumatic force holding unit in a prepared configuration causing reduced likelihood of accidental actuation facilitating patient compliance and treatment outcomes.

Although such devices have provided the advantage of being able to provide improved breath actuated device, there remains room for improvement. Naturally there is a need to develop breath actuated device that is efficient and robust. The present invention improves the robustness of the device by retaining the vacuum within the diaphragm when the mouthpiece cover is left opened for an extended time without being used as discussed herein. The present invention also improves the ease of manufacture by using a simplified structure of the central rigid disk, while improving robustness.

### SUMMARY OF THE INVENTION

The invention is defined by the appended claims.

In one embodiment, the present disclosure provides an improved breath actuated inhaler device comprising an actuator housing, canister and a mechanical release mechanism for triggering the dose in response to breathe. The mechanical release mechanism is similar to that of pneumatic force holding unit disclosed in PCT application WO 01/93933 A2 but includes a structure that is distinct from and also provides substantial improvement. The pneumatic force holding unit comprises a compression spring (preload), a lower cap/ canister support sleeve that engages the canister, a diaphragm attached to an upper surface of the lower cap/ canister support sleeve, and a flap to seal a valve port located in the diaphragm. The diaphragm includes a central rigid disk and a peripheral flexible polymer ring.

In an embodiment, the present disclosure provides an improved breath actuated inhaler device comprising an actuator housing, canister and pneumatic force holding unit comprising a compression spring (preload), a lower cap/ canister support sleeve that engages the canister, a diaphragm attached to an upper surface of the lower cap/ canister support sleeve, and a flap to seal a valve port located in the diaphragm. The diaphragm includes a central rigid disk and a peripheral flexible polymer ring for triggering the dose in response to breathe.

In an embodiment, the present disclosure provides an improved breath actuated inhaler device comprising an actuator housing, canister and pneumatic force holding unit for triggering the dose in response to breathe.

In an embodiment, the present disclosure provides an improved pneumatic force holding unit wherein a central rigid disk of the diaphragm is provided with the interlocking means to firmly secure and bound the peripheral flexible polymer ring.

In an embodiment, the present disclosure provides an improved pneumatic force holding unit wherein a central rigid disk of the diaphragm is provided with the interlocking means to firmly secure and bound the peripheral flexible polymer ring with the central rigid disc of the diaphragm during injection molding of the diaphragm.

The interlocking means are the one or more grooves or cavities. Preferably the interlocking means are between 1 to 25 in numbers, more preferably between 5-20 in numbers. The interlocking means are of same or different geometry and may or may not be located equidistance from each other.

In an embodiment, a central rigid disk and a peripheral flexible polymer ring of diaphragm is formed by two shot injection molding process. In first shot the central rigid disk is prepared having the interlocking means. In second shot a peripheral flexible polymer ring of diaphragm is prepared. During the second shot injection molding process the flexible polymer is allowed to flow through the interlocking means of a central rigid disk to form a continuous uninterrupted part on either side of the central rigid disc of the diaphragm. This allows mechanical bonding of the peripheral flexible polymer ring with the central rigid disc and at the same time creates a strong adhesion with the central rigid disc. Further mechanical interlock between a central rigid disk and a peripheral flexible polymer ring on the diaphragm is prevented from being peeled off from the central rigid disc while the vacuum is being retained prior to inhalation.

In some aspects, the material which can be used to construct the central rigid disk includes Acrylonitrile butadiene styrene (ABS), Polypropylene (PP), Polyethylene (PE), Polytetrafluoroethylene (PTFE). In some aspects, the material which can be used to construct the flexible polymer ring includes Thermoplastic Polyurethane (TPU), Silicone, and Thermoplastic Polyethylene (TPE).

The hardness of the central rigid disk 20 is typically between 80 -140 on R scale of Rockwell hardness but preferably between 100 -120 on R scale of Rockwell hardness. The hardness of the flexible polymer ring is typically between 55-75 Shore A, but preferably between 60-70 Shore A.

In an embodiment, the present disclosure provides an improved pneumatic force holding unit having a diaphragm wherein a central rigid disk of the diaphragm is provided with the interlocking means to firmly secure and bound the peripheral flexible polymer ring with the central rigid disc of the diaphragm during injection molding of the diaphragm, wherein a central rigid disk and a peripheral flexible polymer ring of diaphragm is formed by two shot injection molding process.

In an embodiment, the present disclosure provides an improved pneumatic force holding unit having a diaphragm wherein a central rigid disk of the diaphragm is provided with the interlocking means to firmly secure and bound the peripheral flexible polymer ring with the central rigid disc of the diaphragm during injection molding of the diaphragm, wherein a central rigid disk and a peripheral flexible polymer ring of diaphragm is formed by two shot injection molding process wherein the central rigid disk is prepared having the interlocking means in first shot and a peripheral flexible polymer ring of diaphragm is prepared in second shot during which flexible polymer is allowed to flow through the interlocking means of a central rigid disk to form a continuous uninterrupted part on either side of the central rigid disc of the diaphragm.

In a further aspect of an embodiment, the disclosure provides a flap to seal a valve port of the diaphragm wherein the flap consists of a rigid component and the soft elastomer component wherein a soft elastomer component forms an air tight lock with the valve port of the diaphragm. The hardness of the soft elastomer component is between 10-20 Shore A and preferably between 12-16 Shore A.

In an embodiment, the present disclosure improves the robustness of the device by retaining the vacuum within the diaphragm when the mouthpiece cover is left opened for an extended time without being used, for at least about 5 Minutes, preferably at least about 10 minutes, more preferably at least about 15 minutes, more preferably at least about 25 minutes. The mechanical interlock between a central rigid disk and a peripheral flexible polymer ring on the diaphragm is prevented from being peeled off from the central rigid disc while the vacuum is being retained prior to inhalation. Further the flap forms an airtight seal with the valve port of the diaphragm thereby preventing the leak. When the mouthpiece cover is left opened, the device remained in the actuable condition without firing the dose for at least about 5 minutes, preferably at least about 10 minutes, more preferably at least about 15 minutes, more preferably at least about 25 minutes. The force retained by the pneumatic force holding unit degrades by less than about 6% over a period of 5 minutes, preferably less than about 3%, preferably from about 2.7% to about 1%; 1.5% being an example.

### FIGURES:

Fig. 1 shows a partial-exploded isometric view of the breath-actuated inhaler device.
Fig. 2 shows a partial-exploded isometric view of the breath-actuated inhaler device showing exploded view of pneumatic force holding unit.
Fig. 3 shows an isometric view of diaphragm according to the invention.
Fig. 4 shows an isometric view of a central rigid disk of diaphragm according to the invention.
Fig. 5 shows an isometric view of a peripheral flexible polymer ring of diaphragm according to the invention.
Fig. 6 shows an isometric view of a flap according to the invention.

### DETAILED DESCRIPTION OF THE INVENTION:

The present invention provides a modified version of a diaphragm and a flap for a trigger mechanism of a breath actuated metered dose inhaler and the methods for manufacturing the same.

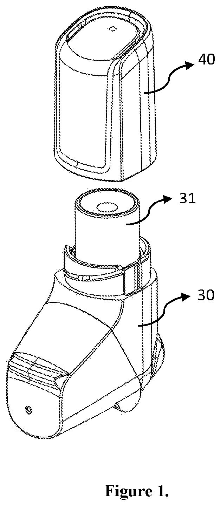

Fig. 1 shows a partial-exploded isometric view of the breath-actuated inhaler device having top housing 40 and bottom housing 30. The inhaler device houses a canister 31 containing a medicament to be dispensed.

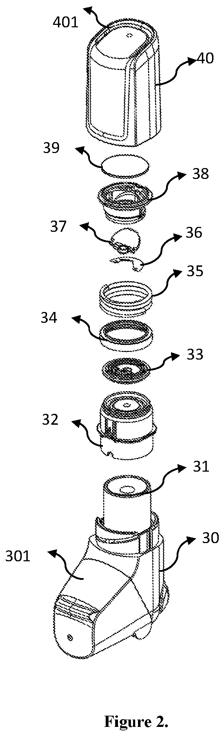

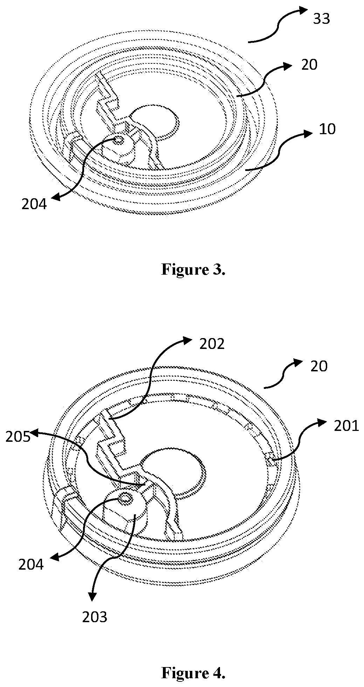

Fig. 2 shows a breath-actuated inhaler device showing exploded view of pneumatic force holding unit. The pneumatic force holding unit showing a canister support sleeve 32, a spring 35, a diaphragm 33, a diaphragm retaining ring 34, a flap 37, a flap spring 36, a flap housing 38, a cloth filter 39. The canister support sleeve 32 encases and drives the canister 31. The spring 35 is in compressed position when the mouthpiece cover 301 is in closed position as can be seen in this figure. The diaphragm 33 is held in place over the canister support sleeve 32 by a diaphragm retaining ring 34 that snaps over the canister support sleeve 32. The spring 35 is a compression spring located between the flange of the flap housing 38 and the flat bearing surface of the diaphragm retaining ring 34. The flap housing 38 bears features to situate the flap component 37 and the flap spring 36. The flap spring 36 biases the flap 37 over the open valve port 204 (Fig. 3) of the diaphragm 33. A cloth filter 39 is welded over the flap housing 38. The pneumatic force holding unit is located inside the top housing 40.

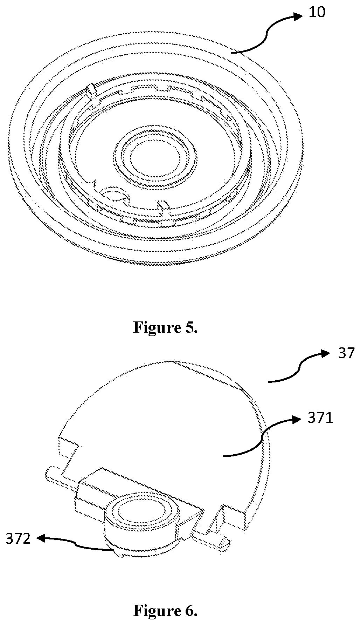

Fig. 3- Fig. 5 shows an isometric view of diaphragm 33 according to the invention having central rigid disk 20 and a peripheral flexible polymer ring 10. Fig. 4 shows an isometric view of a central rigid disk 20 of diaphragm according to the invention and Fig. 5 shows an isometric view of a peripheral flexible polymer ring 10 of diaphragm according to the invention. The central rigid disk 20 has circumferential openings 201, transverse baffle 202, boss 203, valve port 204 and radial rib 205. The transverse baffle 202 helps direct the air flow over the flap 37 (Fig. 2 and Fig. 6) during inhalation. The valve port 204 is closed and sealed by the flap 37 prior to inhalation. The boss 203 surrounding the valve port 204 provides a guided passage for the air to escape during inhalation. The radial rib 205 supports the boss 203 and facilitates the molding process by providing a connection between the boss 203 and the transverse baffle 202, allowing all structures of the central rigid disk 20 to be molded in a single injection shot. The circumferential openings 201 allow the flexible polymer to pass through during the molding process creating a mechanical bond between the two components. The central rigid disk 20 and a peripheral flexible polymer ring 10 of diaphragm is formed by two shot injection molding process. In first shot the central rigid disk 20 is prepared having the circumferential openings 201 which acts as an interlocking means. The circumferential openings 201 are between 1 to 25 in numbers, more preferably between 5-20 in numbers and are of same or different geometry and may or may not be located equidistance from each other. The material which can be used to construct the central rigid disk includes ABS, PP, PE, PTFE. The hardness of the central rigid disk 20 is typically between 80 -140 on R scale of Rockwell hardness but preferably between 100 -120 on R scale of Rockwell hardness. In second shot a peripheral flexible polymer ring 10 of diaphragm is prepared. During the second shot injection molding process the flexible polymer is allowed to flow through the circumferential openings 201 of a central rigid disk 20 to form a continuous uninterrupted part on either side of the central rigid disc 20 of the diaphragm 33. This allows mechanical bonding of the peripheral flexible polymer ring 10 with the central rigid disc 20 and at the same time creates a strong adhesion with the central rigid disc 20. Further mechanical interlock between a central rigid disk 20 and a peripheral flexible polymer ring 10 on the diaphragm 33 is prevented from being peeled off from the central rigid disc 20 while the vacuum is being retained prior to inhalation especially in the extended (stressed) state. The advantage of this particular arrangement is that in addition to the chemical bond created during the two shot molding process, the mechanical bond provides addition security of the two components especially while the vacuum is being retained prior to inhalation especially in the extended (stressed) state. The material which can be used to construct the flexible polymer ring includes TPU, Silicone, TPE. The hardness of the flexible polymer ring is typically between 55-75 Shore A, but preferably between 60-70 Shore A.

Fig. 6 shows an isometric view of a flap according to the invention. The flap 37 consists of a rigid component 371 and the soft elastomer 372. The flap 37 is formed as a two shot part during injection molding process. The soft elastomer 372 forms an air tight seal with the valve port 204 (Fig. 3) of the central rigid disc 20 of the diaphragm 33 when the flap spring 36 is biased on the flap 37. In use, the air flow by a patient causes the flap 37 to bias against the flap spring 36, thereby opening the valve port 204 on the rigid component 20 of the diaphragm 33. The material which can be used to construct the rigid component 371 includes ABS, POM or PTFE. The material which can be used to construct soft elastomer 372 includes TPU, TPE or Silicone. The hardness of the soft elastomer 372 is typically between 5-20 on Shore A, but preferably between 12-16 Shore A to allow enough flexion of the elastomer 372 over the valve port 204.

When in use, once the mouthpiece cover 301 of the bottom housing 30 is opened, the spring 35 extends partially and pushes the canister support sleeve 32 via the diaphragm ring 34. This causes the peripheral flexible polymer ring 10 of the diaphragm 33 to extend. The valve port 204 on the central rigid disc 20 of the diaphragm 33 is closed by the flap 37 biased by the flap spring 36. This prevents the complete extension of the spring 35 due to negative pressure build up between the diaphragm 33 and the canister support sleeve 32. The diaphragm 33 of the present invention is advantageous as it helps in retaining the vacuum in this extended (stressed) state for a longer period due to both chemical bond and mechanical bond created during the two shot molding process. This effect is further amplified and/or maintained by the soft elastomer 372 of a flap 37 which forms an air tight seal with the valve port 204 and thereby preventing vacuum leak. The present invention improves the robustness of the device by retaining the vacuum within the diaphragm 30 and canister support sleeve 32 when the mouthpiece cover is left opened for an extended time without being used, for at least about 5 Minutes, preferably at least about 10 minutes, more preferably at least about 15 minutes, more preferably at least about 25 minutes. The mechanical interlock between a central rigid disk and a peripheral flexible polymer ring on the diaphragm is prevented from being peeled off from the rigid disc while the vacuum is being retained prior to inhalation. When the mouthpiece cover is left opened, the device remained in the actuable condition without firing the dose for at least about 5 minutes, preferably at least about 10 minutes, more preferably at least about 15 minutes, more preferably at least about 25 minutes. The force retained by the pneumatic force holding unit degrades by less than about 6% over a period of 5 minutes, preferably less than about 3%, preferably from about 2.7% to about 1%: 1.5% being an example. When the user inhales, the air enters the device through the vents 401 on the top housing 40. The air flow causes the flap 37 to bias against the flap spring 36, thereby opening the valve port 204 on the central rigid disc 20 of the diaphragm 33 thereby causing a complete vacuum release. This results in complete extension of the spring 35 and the actuation of the canister 31 release of the dose.

Evaluation of the pneumatic force holding unit performance in breath actuated inhalers is done by measuring the ability of the pneumatic force holding unit in retaining a pressure difference after priming over a time testing period, typically 5 minutes. The instrument used was Texture Technologies' Texture Analyzer TA.XTPlus. The widest force probe was attached to the 50kg load cell of the texture analyzer. The pneumatic force holding unit is placed underneath the force probe on the texture analyzer base. The probe was moved downward at a speed of 0.25 mm/s until force reading of 90 N. The probe was retracted 2.6mm above the current position at a speed of 10mm/s. As soon as the probe retracted 2.6mm, the force is recorded as F1. The force probe was allowed to remain in that position for a period of 5 minutes. After 5 minutes have elapsed, the force is recorded as F2. The data was used to calculate the change in force (Delta F (F1-F2)) as well as the percentage change over a period. The results are summarized in Table 1.

**Table 1**

| Pneumatic Force Holding Unit | Test Number | F1 (N) | F2 (N) | Delta F (N) | % Change |
|---|---|---|---|---|---|
| Device 1 | 1 | 27.24 | 27.96 | 0.715 | 2.626% |
| | 2 | 26.95 | 27.49 | 0.539 | 2.000% |
| | 3 | 26.54 | 27.14 | 0.598 | 2.253% |
| Device 2 | 1 | 28.14 | 28.64 | 0.491 | 1.743% |
| | 2 | 28.07 | 28.36 | 0.294 | 1.049% |
| | 3 | 28.01 | 28.48 | 0.471 | 1.681% |
| Device 3 | 1 | 30.30 | 31.10 | 0.794 | 2.620% |
| | 2 | 29.42 | 29.94 | 0.519 | 1.765% |
| | 3 | 28.84 | 29.53 | 0.686 | 2.379% |

As can be seen from the data of table 1, pneumatic force holding unit of breath actuated inhaler according to the present invention improved the robustness of the device by retaining the vacuum within the diaphragm when the mouthpiece cover is left opened for an extended time without being used a patient and thus improves the patient compliance. Surprisingly, such improvements may be achieved even when employing a valve seal (flap) surface having a roughness average that is greater than the 0.15 µm described as critical for retaining pneumatic force in the prior art.

## Claims

1. A breath actuated inhaler device comprising:
an actuator housing (30, 40);
canister (31) and;
a pneumatic force holding unit for triggering the dose in response to breathe;
wherein pneumatic force holding unit comprising a compression spring (35), a lower cap/ canister support sleeve (32) that engages the canister (31), a diaphragm (33) attached to an upper surface of the lower cap (32), and a flap (37) to seal a valve port (204) located in the diaphragm (33),
wherein the diaphragm (33) includes a central rigid disk (20) and a peripheral flexible polymer ring (10) for triggering the dose in response to breathe,
wherein the central rigid disk (20) of the diaphragm (33) is provided with an interlocking means (201) to firmly secure and bound the peripheral flexible polymer ring (10) with the central rigid disc (20) of the diaphragm (33);
wherein the central rigid disk (20) and the peripheral flexible polymer ring (10) of the diaphragm (33) is formed by a two shot injection molding process wherein the central rigid disk (20) is prepared having the interlocking means (201) in a first shot of the two shot injection molding process and the peripheral flexible polymer ring (10) of the diaphragm (33) is prepared in a second shot of the two shot injection molding process during which flexible polymer is allowed to flow through the interlocking means (201) of the central rigid disk (20) to form a continuous uninterrupted part on either side of the central rigid disc (20) of the diaphragm (33).

2. The breath actuated inhaler device according to claim 1, wherein the flap (37) consists of a soft elastomer component to form an air tight lock with the valve port (204) of the diaphragm (33).

3. The breath actuated inhaler device according to claim 1, wherein the hardness of the flexible polymer ring (10) is between 55-75 Shore A.

4. The breath actuated inhaler device according to claim 2, wherein the hardness of the soft elastomer component is between 5-20 Shore A.

## Patentansprüche

1. Atmungsbetätigte Inhalatorvorrichtung, umfassend:
ein Aktorgehäuse (30, 40);
Behälter (31) und;
eine pneumatische Krafthalteeinheit zum Auslösen der Dosis als Reaktion auf das Atmen;
wobei die pneumatische Krafthalteeinheit eine Druckfeder (35), eine untere Kappen-/Behälterstützhülse (32), die mit dem Behälter (31) in Eingriff steht, eine Membran (33), die an einer oberen Oberfläche der unteren Kappe (32) befestigt ist, und eine Klappe (37) zum Abdichten einer Ventilöffnung (204), die sich in der Membran (33) befindet, umfasst, wobei die Membran (33) eine zentrale starre Scheibe (20) und einen peripheren flexiblen Polymerring (10) zum Auslösen der Dosis als Reaktion auf das Atmen beinhaltet, wobei die zentrale starre Scheibe (20) der Membran (33) mit einem
Verriegelungsmittel (201) versehen ist, um den peripheren flexiblen Polymerring (10) mit der zentralen starren Scheibe (20) der Membran (33) fest zu sichern und zu binden;
wobei die zentrale starre Scheibe (20) und der periphere flexible Polymerring (10) der Membran (33) durch ein Zwei-Schuss-Spritzgussverfahren gebildet werden, wobei die zentrale starre Scheibe (20) mit dem Verriegelungsmittel (201) in einem ersten Schuss des Zwei-Schuss-Spritzgussverfahrens hergestellt wird und der periphere flexible Polymerring (10) der Membran (33) in einem zweiten Schuss des Zwei-Schuss-Spritzgussverfahrens hergestellt wird, während dessen ermöglicht wird, dass flexibles Polymer durch das Verriegelungsmittel (201) der zentralen starren Scheibe (20) fließt, um einen kontinuierlichen ununterbrochenen Teil auf beiden Seiten der zentralen starren Scheibe (20) der Membran (33) zu bilden.

2. Atmungsbetätigte Inhalatorvorrichtung nach Anspruch 1, wobei die Klappe (37) aus einer weichen Elastomerkomponente besteht, um eine luftdichte Verriegelung mit der Ventilöffnung (204) der Membran (33) zu bilden.

3. Atmungsbetätigte Inhalatorvorrichtung nach Anspruch 1, wobei die Härte des flexiblen Polymerrings (10) zwischen 55 und 75 Shore A liegt.

4. Atmungsbetätigte Inhalatorvorrichtung nach Anspruch 2, wobei die Härte der weichen Elastomerkomponente zwischen 5 und 20 Shore A liegt.

## Revendications

1. Dispositif d'inhalateur actionné par la respiration, comprenant :
un boîtier d'actionneur (30, 40) ;
une cartouche (31) et ;
une unité de maintien de force pneumatique destinée à déclencher la dose en réponse à la respiration ;
ladite unité de maintien de force pneumatique comprenant un ressort de compression (35), un manchon de support de capuchon inférieur/cartouche (32) qui s'engage dans la cartouche (31), un diaphragme (33) fixé à une surface supérieure du capuchon inférieur (32), et un volet (37) permettant de sceller un orifice de soupape (204) situé dans le diaphragme (33), ledit diaphragme (33) comprenant un disque rigide central (20) et un anneau polymère flexible périphérique (10) pour déclencher la dose en réponse à la respiration, ledit disque rigide central (20) du diaphragme (33) étant muni d'un moyen de verrouillage (201) pour fixer fermement et lier l'anneau polymère flexible périphérique (10) au disque rigide central (20) du diaphragme (33) ;
ledit disque rigide central (20) et ledit anneau polymère flexible périphérique (10) du diaphragme (33) étant formés par un processus de moulage par injection à deux passes dans lequel le disque rigide central (20) est préparé avec le moyen de verrouillage (201) dans une première passe du processus de moulage par injection à deux passes et ledit anneau polymère flexible périphérique (10) du diaphragme (33) étant préparé dans une seconde passe du processus de moulage par injection à deux passes au cours duquel le polymère flexible est autorisé à s'écouler à travers le moyen de verrouillage (201) du disque rigide central (20) de manière à former une partie ininterrompue continue de chaque côté du disque rigide central (20) du diaphragme (33).

2. Dispositif d'inhalateur actionné par la respiration selon la revendication 1, ledit volet (37) étant constitué d'un composant élastomère souple pour former un verrou étanche à l'air avec l'orifice de soupape (204) du diaphragme (33).

3. Dispositif inhalateur actionné par la respiration selon la revendication 1, ladite dureté de l'anneau polymère flexible (10) étant comprise entre 55 et 75 Shore A.

4. Dispositif inhalateur actionné par la respiration selon la revendication 2, ladite dureté du composant élastomère souple étant comprise entre 5 et 20 Shore A.
